(19) Europäisches Patentamt
European Patent Office
Office européen des brevets

(11) **EP 2 279 490 B1**

(12) **EUROPEAN PATENT SPECIFICATION**

(45) Date of publication and mention of the grant of the patent:
**21.03.2012 Bulletin 2012/12**

(51) Int Cl.:
*G06T 7/00* (2006.01)    *G06T 7/60* (2006.01)
*A61B 6/00* (2006.01)

(21) Application number: **09732057.6**

(22) Date of filing: **16.04.2009**

(86) International application number:
**PCT/IB2009/051588**

(87) International publication number:
**WO 2009/128042 (22.10.2009 Gazette 2009/43)**

(54) **AUTOMATIC DETECTION AND ACCURATE SEGMENTATION OF ABDOMINAL AORTIC ANEURYSM**

AUTOMATISCHE DETEKTION UND GENAUE SEGMENTIERUNG DES BAUCHAORTENANEURYSMA

DÉTECTION AUTOMATIQUE ET SEGMENTATION PRÉCISE D'UN ANÉVRISME DE L'AORTE ABDOMINALE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **16.04.2008 US 45283 P**

(43) Date of publication of application:
**02.02.2011 Bulletin 2011/05**

(73) Proprietor: **Université de Lausanne**
**1015 Lausanne (CH)**

(72) Inventors:
• **QANADLI, Salah**
  **CH-1009 Pully (CH)**
• **DEHMESHKI, Jamshid**
  **London SW15 2QE (GB)**
• **AMIN, Hamdan**
  **London HA7 1DA (GB)**

(74) Representative: **Schmauder & Partner AG**
**Patent- & Markenanwälte VSP**
**Zwängiweg 7**
**8038 Zürich (CH)**

(56) References cited:
**US-A1- 2006 280 351**

• **DEHMESHKI J ET AL: "Computer Aided Detection and Measurement of Abdominal Aortic Aneurysm Using Computed Tomography Digital Images" DIGITAL SOCIETY, 2009. ICDS '09. THIRD INTERNATIONAL CONFERENCE ON, IEEE, PISCATAWAY, NJ, USA, 1 February 2009 (2009-02-01), pages 339-342, XP031424637 ISBN: 978-1-4244-3550-6**
• **ZHUGE FENG ET AL: "An abdominal aortic aneurysm segmentation method: Level set with region and statistical information" MEDICAL PHYSICS, AIP, MELVILLE, NY, US, vol. 33, no. 5, 28 April 2006 (2006-04-28), pages 1440-1453, XP012092112 ISSN: 0094-2405**
• **KANITSAR A ET AL: "CPR - curved planar reformation" VIS 2002. IEEE VISUALIZATION 2002. PROCEEDINGS. BOSTON, MA, OCT. 27 - NOV. 1, 2002; [ANNUAL IEEE CONFERENCE ON VISUALIZATION], NEW YORK, NY : IEEE, US, 1 November 2002 (2002-11-01), pages 37-44, XP031212945 ISBN: 978-0-7803-7498-0**
• **BULPITT A J ET AL: "SPIRAL CT OF ABDOMINAL AORTIC ANEURYSMS: COMPARISON OF SEGMENTATIONWITH AN AUTOMATIC 3D DEFORMABLE MODEL AND INTERACTIVE SEGMENTATION" PROCEEDINGS OF THE SPIE - THE INTERNATIONAL SOCIETY FOR OPTICAL ENGINEERING, SPIE, PO BOX 10 BELLINGHAM WA 98227-0010 USA, vol. 3338, no. PART 01/02, 23 February 1998 (1998-02-23), pages 938-946, XP008001176 ISSN: 0277-786X**

**(Cont. next page)**

- **SUBASIC M ET AL: "Model-based quantitative AAA image analysis using a priori knowledge" COMPUTER METHODS AND PROGRAMS IN BIOMEDICINE, ELSEVIER, AMSTERDAM, NL, vol. 80, no. 2, 1 November 2005 (2005-11-01), pages 103-114, XP025297011 ISSN: 0169-2607 [retrieved on 2005-11-01]**

## Description

Technical field

This invention concerns an efficient algorithm for automatic detection and accurate segmentation of Abdominal Aortic Aneurysm (AAA). The algorithm first identifies the location of the lumen (the inner portion of aorta) and then segments it. The abdominal portion of the lumen is then found using anatomical and geometrical features. This portion of the lumen is straightened using geometrical transformation based on the smoothed centreline. The transformed lumen is then passed through a number of filters, based on geometrical, intensity, gradient and texture features, to search for the existence of the aneurysm. If aneurysm is detected, a deformable model is first initialized to the approximate borders of the aneurysm which are then refined using global and location information

Background art

An Abdominal Aortic Aneurysm (AAA) is a localised dilation (swelling or enlargement) of an aorta. An AAA usually consists of two sections - the lumen (the inner part) and the thrombus (the outer fatty part). Blood flows in the lumen and its visibility can be enhanced when CT Angiography (CTA) is used. The progressive growth of an aneurysm may eventually cause a rupture if not diagnosed or treated. This can be life threatening as the rupture would cause massive internal bleeding. The probability of a rupture occurring depends on its size. For example, patients exhibiting an AAA of 5 cm or more in diameter should be treated to replace the weakened section by open surgery or using a stentgraft (endovascular procedure). For a 4 cm AAA, if the aneurysm increases by 5 mm or more in six months, treatment should be considered. As a follow-up after the operation, a frequent life-long monitor will be used to ensure that no further expansion has occurred to prevent the chance of further ruptures.

In England and Wales, there are as many as 6,000 to 10,000 people who suffer from an AAA rupture each year; most of whom are men aged 65 and above [1]. In general, about 1 in 20 people in the UK over the age of 65 develop an aortic aneurysm [2]. In USA, as many as 200,000 people are diagnosed each year with the mortality rate of about 15,000 annually [3]. Thus, without doubt, the quantification of aneurysms in CT scans plays a significant role in monitoring the disease both before and after surgery.

Typically, CTA is conducted by injecting patients with a contrast agent to enhance the blood stream in the CT images. The radiologist then manually identifies the enlarged portions of the aorta on a number of cross-sectional images in order to obtain a full volume measure.

This extremely tedious and time-consuming process may take up to 30 minutes and is inconvenient for physicians. Furthermore, this approach becomes impractical as the datasets produced by the latest CT scan machines increase. In addition, such manual methods are subjective, prone to error and non-reproducible. In fact, the validity of the method is questionable as different measurements can arise for the same aneurysm region when performed by different radiologists or by the same radiologist at different times.

The detection and accurate segmentation of an AAA region is a challenging task since it is very difficult, though not impossible, to identify and differentiate between the boundaries of an AAA and the surrounding muscles or other vascular structures. There are a few research publications on computerized AAA segmentation from which computerized measurements can be achieved.

Reference [6] introduces a five-part energy function defined on polar coordinates using a snake method and validates the segmentation result based on 34 ultrasound images.

References [7-8] provide studies of various approaches to segmenting both the aneurysm and the aortic flow channel employing a level set framework using either edge strength or region intensity information. These methods are not fully automatic and require one or several external seed points for initialisation. Further, they have not been robustly validated.

There exist on the market a number of Computer Aided Detection (CAD) systems using image processing analysis that assist radiologists in detecting abnormalities in medical imaging more efficiently. The medical image processing methods are highly application oriented which means that different algorithms are developed to cater for different parts of human body such as the lung (CAD from Siemens [4b]), breast (GE-Mammography [5b]) and others.

The members of Mediar Ltd. have in the past designed and produced several scientific image processing algorithms, each developed for a specific medical imaging application. Some of the publications are provided in references [6b] to [10b]. In [6b] a description of an image processing algorithm is provided for the quantification of calcium plaque in heart using the Modified Expectation Maximization (MEM) method. Reference [7b] tackles the segmentation problem in brain from MRI images using Multiple Discriminant Analysis (MDA). References [8b] and [9b] present CAD systems in colon using several image processing methods such as fuzzy connectivity, shape index and clustering analysis. Reference [10b] gives a description of lung nodule segmentation using a shaped based region growing algorithm.

Despite the growing importance of having a dynamic approach that would help radiologists obtain accurate measurements of the Abdominal Aortic Aneurysm (AAA), there is no real computerized image processing analysis system commercially available. This is because the task of extracting the AAA region from the CT scans and measuring it accurately by a computerized image analysis system is problematic. This is due to the fact that the AAA boundaries often touch muscles and other tissues of similar intensities making it very difficult, though not impossible, to identify and differentiate between the boundaries of the adjacent tissues.

There is accordingly an immediate demand for a new and sophisticated image processing approach that uses scientific algorithms to effectively extract any AAA regions and produce precise measurements.

Although there are a few academic papers that have attempted to tackle this problem (see references [11b] to [20b]), they are not robust enough to be considered for future commercial use.

Reference [11b] introduces a five-part energy function defined on polar coordinates using a snake method and validates the segmentation result based on 34 ultrasound images.

References [12b-13b] provide studies of various approaches to segmenting both the aneurysm and the aortic flow channel employing a level set framework using either edge strength or region intensity information. Further, they have not been robustly validated. These methods are not fully automatic and require one or several external seed points for initialisation.

In reference [9], an active shape model (ASM) is used to segment the AAA. In this approach, the user has to draw a two-dimensional (2-D) contour on one slice which is propagated to the adjacent slice based on gray values similarity. The optimal fit is defined by maximum correlation of gray value profiles around the contour in successive slices.

In reference [10], an interactive AAA segmentation system is developed based on the active shape model. If an obtained contour is not sufficiently accurate, the user can intervene and provide an additional manual reference contour. Although accurate, the amount of user interaction is large, because slice-by-slice user-interaction is required. Further, the result may vary considerably with different attempts.

Reference [11] investigates a 3D active shape model method with a gray level appearance model based nonparametric classification technique for AAA segmentation. The method requires manually drawn top and bottom slices and a user input point in the approximate aneurysm centre of the central slice.

Another method is described in reference [12], where a level-set algorithm is used to segment the aneurysm from an initial region which is drawn as a sphere by the user. The sphere is deformed to identify the borders of the AAA. Although user interaction is minimal, the reported results are not accurate.

In reference [13], a method is described for the aortic aneurysm segmentation using a constrained contour evolution method. At each iteration, the snake points are driven by a rejecting force that depends on the difference between local Hounsfield Unit (HU) value and thrombus HU and an edge force directed along the derivative of the image gradient that is active only if the edge shape is compatible with the border of a thrombotic region. User interaction is vital to achieve good results.

Another method is presented in reference [14]. This method is based on a deformable model called a 3D active object. First, the algorithm segments the lumen (the inner portion of aorta), which is then used to initialize the segmentation of the AAA (the rest of the expanded fatty portion). A nonparametric gray level appearance model that is based on the intensity profile is used to drive the deformable model. Accurate results with less required user intervention were reported. However, a parameter estimation using a classifier is required for different datasets which calls for manual segmentation of AAA boundaries for a subset of the data. Once the system is trained, the user provides two seeds on the same slice and the algorithm extracts the AAA. This algorithm was only tested on pre-operative cases of a total of 17 patients.

Another method in AAA segmentation is presented in reference [15]. This method estimates a rough initial surface, and then refines it by using a level set segmentation scheme augmented with a global region and a local feature analyzer. One drawback is that the deformable model segmentation assumes that the aneurysm is roughly circular in a transaxial cross section, thus resulting in failure of the segmentation for non-circular shaped aneurysm. The system was only tested on 20 CTA AAA datasets.

Other prior art publications include WO 03/075209, WO 2004/081874 and US 2006/025674.

Reference [20b], i.e.: F. Zhuge et al., Med. Phys. 33(5), May 2006, p. 1440-1453, discloses a computer-implemented, automated method of segmenting computed tomography angiography (CTA) images comprising the steps of: (i) receiving said CTA images, identifying the lumen of an aorta in the received images, and extracting said lumen, (ii) searching for features of said straightened abdominal portion that indicate the presence of an aneurysm, and (iii) if the presence of an aneurysm is detected based on said features, segmenting the aneurysm using said extracted lumen as an initial surface of a deformable model.

Kanitsar et al., Proc. IEE Visualization 2002, Oct. 27 - Nov. 1, 2002, Boston, MA, USA, p. 37 - 44, disclose the steps of identifying the abdominal portion of said extracted lumen in said CTA images and performing a straightening of said abdominal portion to obtain a straightened abdominal portion.

Summary of the invention

As the reliability of the measurement depends on how accurately the regions of interest can be segmented, an aim of the present invention is to improve the measurement methods and systems of the prior art.

More specifically, an aim of the invention is to concentrate on the development of an automatic and accurate segmentation of AAA as a first and essential stage for accurate measurement.

In comparison to the manual identification, segmentation and measurement of AAA, the proposed system and method offers more accurate, reproducible and cost-effective, plus faster results.

Brief description of the drawings

Figure 1 illustrates an embodiment of the overall system and method according to the present invention;

Figures 2(a) to 2(d) illustrates the detection and extraction of the lumen: (a) Original sagittal view, (b) 3D view of the segmented image, (c) 3D view of morphological operation on (b),(d) the extracted lumen wherein the white arrows point to the lumen;

Figure 3 is a 3D view of a lumen where the diaphragm arch is added artificially;

Figures 4(a) to 4(c) represent lung regions: (a) Coronal view showing abdominal aorta at the lung regions, (b) axial view of a lung, (c) extracted holes of the segmented lung;

Figures 5(a) to 5(c) represent a Celiac Trunk: (a) sagittal view showing the Celiac Trunk (arrowed), (b) Projected sagittal view of a segmented aorta, (c) morphological operation on (b) where the top isolated object is the celiac trunk;

Figures 6(a) to 6(c) illustrate a geometrical transformation (3D views of the segmented lumen); a) original lumen, b) geometrically transformed lumen, c) overlap between a and b, the original position is highlighted with an arrow;

Figures 7(a) and (b) illustrate examples of the presence of aneurysm; the bottom images are the projection of coronal view of segmented aorta and the top images show the slices indicated by the white arrows; only the segmented lumen are shown in the bottom images: (a) lumen is bent and has large diameter, (b) lumen is straight with small diameter but irregular lumen cross section

Figures 8(a) and (b) illustrate fat regions, (a) original image, (b) segmented fat regions, 3D view of the segmented fat regions;

Figures 9(a) to (c) illustrate a method of filling the gap between bone edges of a spine where the top images are axial and the bottom images are sagittal views; (a) original image, (b) segmented fat and spine, (c) similar to (b) with gaps filled (arrowed) between the bone edges

Figures 10(a) and (b) are examples of blood vessel next to an aorta: (a) the 3D image clearly shows the difference in geometrical features between the blood vessel and the calcified region, no thrombus is shown in the 3D image, (b) the result shows that the blood vessel are retained as part of non-AAA regions;

Figure 11 illustrates the process of searching for aneurysm from the features extracted from the Potential Regions (PRs). The global filter uses the results of the rule-based filters from each PR with different lumen expansion;

Figures 12 illustrates examples of different lumen expansions and obtaining the corresponding attached regions (PR): in the top row, there is aneurysm whilst the in bottom row there in no aneurysm;

Figures 13(a) to (f) illustrate an Ellipsoid approximation, (a) non_AAA mask where the white areas are the non_AAA regions, (b) distance map on (a), (c) 70% core region of (b), (d) left region is the zoomed out of (c) and the right region is the result of opening operation of the left region, (e) expanding (d) to hit the edges of the non_AAA, f) same region in (e) shown on the original image;

Figure 14 shows a table of distances between end of lung and the Celiac Trunk for 40 CTA images using the parameters $T_{size}=6$, $T_{cnt}=6$;

Figure 15 shows examples of the segmentation of AAA

Detailed description of the invention

According to the present invention, a computer-implemented, automated method of segmenting computed tomography angiography (CTA) images comprises the following steps:

(1) receiving said CTA images, identifying the lumen of an aorta in the received images, and extracting said lumen, this step comprising the following sub-steps:

a. segmenting said CTA images using threshold limits that represent the intensities of the contrast agent intensity range to obtain segmented CTA images;
b. performing a morphological erosion operation to break connections between 3D objects in said segmented CTA images;
c. applying a geometrical filter to identify the lumen among a plurality of 3D objects;

(2) identifying the abdominal portion of said extracted lumen in said CTA images;
(3) performing a straightening of said abdominal portion to obtain a straightened abdominal portion;
(4) searching for features of said straightened abdominal portion that indicate the presence of an aneurysm;
(5) if the presence of an aneurysm is detected based on said features, segmenting the aneurysm using said extracted lumen as an initial surface of a deformable model.

Each of the abovementioned steps is described in more detail below.

**1) Identify and extract lumen:**

Lumen identification is achieved through gathering prior knowledge of their shape, appearance and geometrical representations in relation to other tissues. Anatomically, the aorta (lumen) runs alongside the spine before branching off into the two main arteries that run down the legs. Further, the enhanced intensity level of lumen in the CT Angiography images assists in locating the lumen more accurately. The identification and extraction of the lumen is done as follow:

a. Segment the CTA image using threshold limits that represents the intensities of the contrast agent intensity range.
b. Perform morphological erosion operation to break the weak links between 3D objects in the segmented image.
c. Apply a geometrical filter to identify the lumen among many 3D objects. As regards to this, the lumen is relatively long in Z direction and narrow (in coronal view), plus it resides approximately in the middle of the body.

**2) Identify the abdominal portion of the lumen:**

The abdominal portion of the lumen is located between the Celiac Trunk and Iliac Arteries junction. The Celiac Trunk is the first artery that stems out of the lumen below the diaphragm which is approximately where the lung ends. The Iliac Arteries junction is where the lumen is split into two major arteries. The algorithm for identifying the abdominal portion of the lumen is outlined below:

a. Find the end location of the lung region by using the following image processing algorithms slice by slice until no more lung region is found:

i. Perform an adaptive fuzzy thresholding segmentation algorithm.
ii. Extract the possible lung regions by finding holes within the segmented image; the hole extraction is done by applying the flood-fill algorithm on the segmented image and subtracting the result from the segmented image.
iii. Check if the region, obtained in Step 2a_ii, contains several small holes; the small holes are the result of taking the cross sectional view of many blood vessels within the lung space.
iv. If lung region is identified in Step 2a_iii, then go to Step 2a_i examining the next slice; otherwise the current slice is the end location of the lung regions.

b. Find Celiac Trunk using the end of lung location (from Step 2a) and the extracted lumen (from Steps 1).

i. Create a sub-image as a search volume whose depth is limited to soma above and 100mm below the end of lung location. The axial dimension of the sub-image is obtained by taking a 10% enlargement of the rectangle that encompasses the lumen that was obtained in Step 1.

ii. Segment the sub-image based on Step 1a and keep the region that intersects with the lumen (Step 1c).

iii. Project all voxels in the sagittal view into one slice.

iv. Perform the erosion operation of a window size 15x15 followed by the dilation operation of 19x 19 window size.

v. Subtract image in Step 2b_iv from the image in Step 2b_iii. This will result in several isolated objects that represent the branches of the lumen.

vi. Get the first isolated object from the left and top as the first branch of the lumen, which is the Celiac Trunk.

c. Find Iliac Arteries junction using smoothed centreline and cluster connectivity analysis.

i. Get the centreline using the Distant Transform-based Skeletonization method.

ii. Smooth the centreline by using an iterative method in which the current location of a voxel within the centreline is updated by the weighted average of deviation vector of its neighbour voxels.

iii. Create the cluster connectivity class of the centreline

iv. Identify the main branches (Iliac Arteries) by evaluating the length of each branch (from the cluster connectivity class). The length of other branches (arteries) is relatively short.

## 3) Geometrical Transformation

Using the centreline in Step 2c and the location of the Celiac Trunk and Iliac Arteries (Step 2b and 2c) as end points, the centreline is straightened by finding the shortest path between the two end points. The geometrical mapping that is obtained from the centreline transformation can be applied on the segmented or the original image. This way, all features collected from the lumen and the surrounding voxels will be virtually taken from the planes orthogonal to the original position of the lumen.

## 4) Search for aneurysm features

The geometrically transformed lumen is first examined for the indication of the aneurysm and then the objects attached to the lumen are evaluated. The search for aneurysm is presented below:

a. Check if the lumen is bent: An amount of the geometric transformation in Step 3 would provide an indication of how much the lumen is moved to one side.

b. Check if the diameter of the lumen is enlarged: The diameter of a normal lumen should be about 30mm.

c. Check if the cross section of the lumen is non-circular: Apply compactness and circularity measure.

d. Check if the attached objects to the lumen have features of aneurysm: To get the attached objects, first the non-AAA regions are obtained. These regions have some intensity and geometrical features that are very different from that of the aneurysm; therefore they can be safely and easily identified. The search for aneurysm among the attached objects is outlined below:

i. Get the non-AAA - fat regions: Any region whose intensity is below -10 and whose volume is greater than $500mm^3$ is considered to be part of the fat regions. The volume threshold is used to exclude the image artefacts.

ii. Get the non-AAA - high intensity objects: All objects that were discarded in Step 1c (such as spine and kidneys) are added to the non-AAA regions.

iii. Get the non-AAA - spinal bone gap filling: The spinal disks have intensity values that are similar to that of the aneurysm. This leads to the formation of openings in the slices where the disk regions touch the borders of the aneurysm. A geometrical interpolation between the edges of the spinal bones in the consecutive slices is done to fill the gap.

iv. Extract attached objects: The attached objects are obtained by subtracting the lumen from its expansion and finding the intersection of this result with the inverse of non-AAA regions. This mask is then used to obtain the intensity, gradient and texture features from the original image. Note that the voxels of the original image are interpolated based on the geometrical transformation map (from Step 3). The extracted features are fed to a rule-based filter to search for the existence of the aneurysm. The characteristics of the rule-base filter are found by gathering and evaluating a statistical analysis of the features of known regions (classified as AAA or non-AAA by radiologists) during experimentation.

**5) Segment aneurysm**

The extracted lumen (Step 2) is used as an initial surface and the non-AAA regions (Step 4d_i and 4d_ii) are used as barriers for a deformable model based segmentation.

a. The AAA region is first approximated by robustly fitting a 3-D ellipsoid anisotropic Gaussian-based intensity model to the border of the non-AAA regions. A union of this region and the extracted lumen is used as an initial region to be refined at its border; this is given in the next step.

The deformable model, initialised in the previous step, is driven by the global and local information. The global information uses the graphical representations of anatomical structures and relationships that constitute the human body, whilst the local information utilises the 3D geometric features calculated at each voxel as a measure of local shape information.

The method is described in a more detailed manner in the following with reference to the drawings.

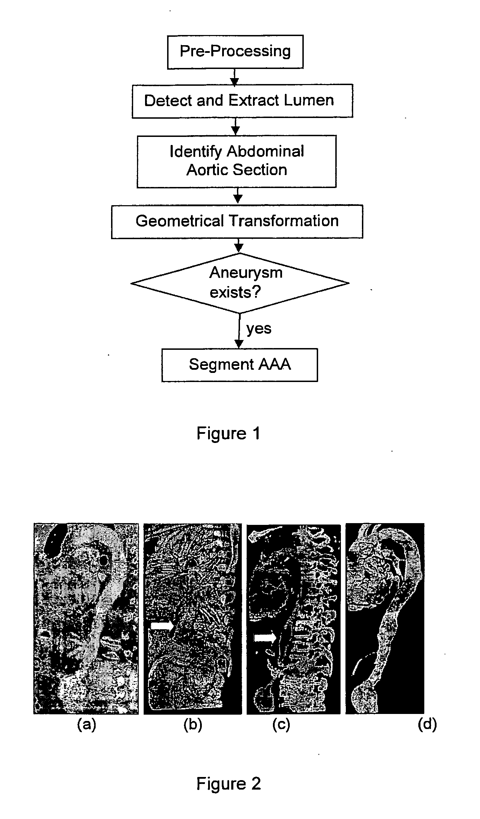

Figure 1 shows the overall design of the automatic and accurate segmentation of AAA.

The CTA image is first smoothed using the anti-geometric diffusion method in the pre-processing stage. Lumen is then extracted from the pre-processed image using segmentation and morphological operation. The abdominal portion of the lumen is identified using geometrical information, mathematical morphology and connectivity cluster analysis. This is followed by the geometrical transformation to straighten the lumen using its centreline. Several evaluations are then carried out on the abdominal aortic section to search for the presence of aneurysm. If aneurysm is identified, the full segmentation of the AAA will follow.

**1) Detect and Extract Lumen**

Accurate segmentation of lumen is not necessary since lumen will be used as an initial region to detect and segment the aneurysm. The segmentation of the lumen is performed using a threshold-based segmentation with low and high threshold values of $T_l$ and $T_h$; these threshold limits were found experimentally to be 140 and 700 respectively. Due to the partial volume effect, the extracted lumen often has loose connection with objects of similar HU attenuation such as spine and kidneys through renal arteries. To separate lumen from other objects, the morphological erosion operation is applied which will result in several isolated 3D objects. For further elimination of the loose links, a special 3D labelling algorithm is applied whereby the core regions of the cross sections in the consecutive slices are used to identify the existence of the connectivity. The core of a region is obtained by first taking the distance transform and finding the centre point (maximum distance value). The region core is then identified as a blob that contains the centre point as well as all the points that have distance value of some percentage of the maximum distance. A 70% region core was used in this experiment.

The morphological operation and the special 3D labelling results in several isolated 3D objects. Among these isolated objects, the lumen can be identified as the object that is relatively long in Z direction and narrow (in coronal view), plus it resides approximately in the middle of the body.

Figure 2 shows the effect of the erosion operation on separating the lumen from other objects. The labelled 3D view in Figure 2c illustrates that the erosion operation has completely separated the lumen.

**2) Identifying Abdominal Lumen Section**

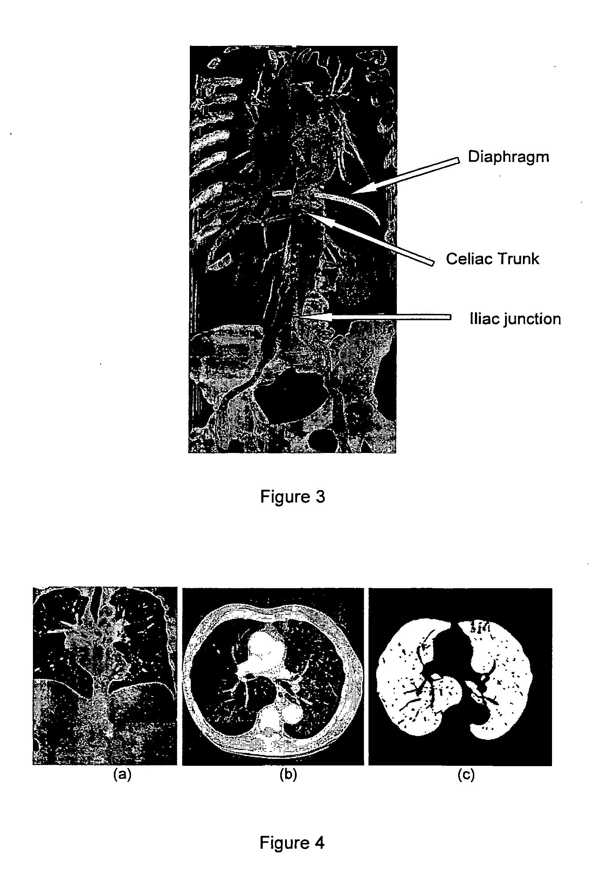

The abdominal section of the lumen is identified automatically by finding the positions of the Celiac Trunk and the Iliac Arteries junction as shown in Figure 3. The Celiac Trunk is located after the diaphragm which is approximately where the lung ends; the diaphragm arch is artificially drawn in Figure 3 to indicate its approximate location in the human body.

**2.1) End of Lung**

The first step in identifying the abdominal portion of the lumen is to find the end location of the lung regions. Lung regions appear as hollowed objects in the CT images due to the presence of large volume of air, as shown in Figure 4b. Using the fuzzy-threshold segmentation together with the morphological hole-extraction algorithms, lung regions can be extracted. The hole-extraction algorithm is performed by flood-filling the segmented body region and then subtracting it from the original segmented body. The holes will then appear as isolated objects.

The lung regions, extracted as hollowed objects, contain a number of smaller holes which are the result of cross sectional views of many blood vessels in the lung regions. The cross section of a segmented lung in Figure 4c indicates the presence of many small holes. The identification of lung objects can thus be carried out by counting the number of the holes of a considerable size within the hollowed regions; i.e. tiny holes are not considered because they might be due to noise. Let $O_k$ be the *kth* extracted hollowed object in the current slice, then $O_k$ is part of the lung region ($O_k \in L$) if the following condition holds,

$$if([\sum_{p=0}^{N} S\ (O_k(p))] > T_{cnt})\quad then\quad O_k \in L \tag{1}$$

Where N is the total number of isolated objects (holes) inside the *Kth* object $O_k$, $O_k(p)$ is the *pth* isolated object, $T_{cnt}$ is an object count threshold, and

$$S\ (x) = \begin{cases} 1 & if\ (size(x) > T_{size}) \\ 0 & otherwise \end{cases} \tag{2}$$

Where $T_{size}$ is the object size. Experimentally $T_{size}$ = 5 and $T_{cnt}$ =10 were chosen.
This process of identifying the lung regions is continued slice-by-slice until no lung region is found. This will be the approximate location of the diaphragm or the end of the lung regions.

### 2.2) Celiac Trunk

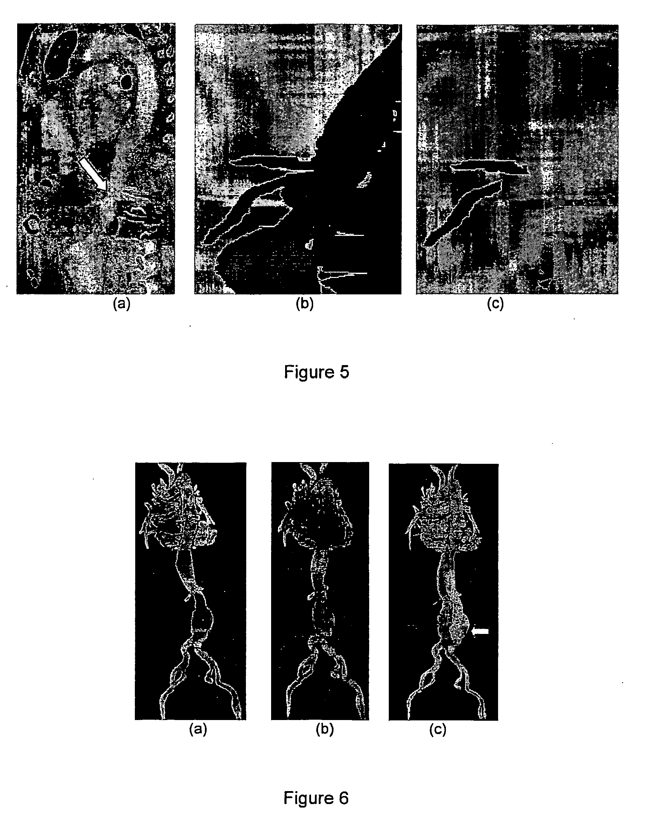

The end location of the lung regions only provides an approximate position where the search for the Celiac Trunk can be carried out. The search volume will be within a volume of an enlarged lumen (20% enlargement) and limited to 20mm above and below the end of lung location. By using an adaptive segmentation, a new lumen is obtained with the search volume to ensure that a fully detailed lumen is obtained; note that the previously extracted lumen lack detail due to the morphological operation. A projected sagittal view of the segmented aorta is then obtained which is shown in Figure 5b. Two morphological operations of erosion and dilation, with window size of the dilation operation larger than that of the erosion operation, will result in separating the branches from the main lumen; this is given in Figure 5c. Since the Celiac Trunk is the first branch-off from the lumen, it will be the top object after the morphological operation as indicated in Figure 5c.

### 2.3) Iliac Arteries Junction

Iliac Arteries junction is the last location to be found for the abdominal aortic identification. This is where the aorta splits into two major arteries running down the legs. To identify this location, the centre-line of the aorta is first obtained then is passed through a smoothing filter followed by a conversion to the cluster connectivity. Using this cluster connectivity, a cluster analysis based on length and angle is used for every branches stemming-out from the main centre-line (the lumen). As regard to this, the length of all branches, except for the Iliac arteries, is relatively short and their angle with respect to the main centre-line is relatively sharp.

### 3) Geometrical Transformation

The abdominal portion of the lumen is geometrical transformed to straighten the lumen by using its 3D centreline. The reason for the transformation is to ensure that the subsequent feature extraction would represent the information taken from the plain orthogonal to the lumen's original centreline. Figure 6 shows the result of the transformation of a lumen. For better visualisation, the original and the transformed lumen are combined in the 3D image of Figure 6c.

### 4) Detecting Aneurysm

Several features are used to detect the presence of aneurysm. First, the features from the extracted lumen are obtained and evaluated. If no aneurysm is detected, then features from the raw image are used for further evaluation. These features are outlined as follows:

1. Check if the diameter of the lumen is enlarged
2. Check if some of the lumen's cross sections are irregular (non-circular)
3. Check if the lumen is displaced (not straight)

4. Check if the attached objects to the aorta contain predefined features for aneurysm

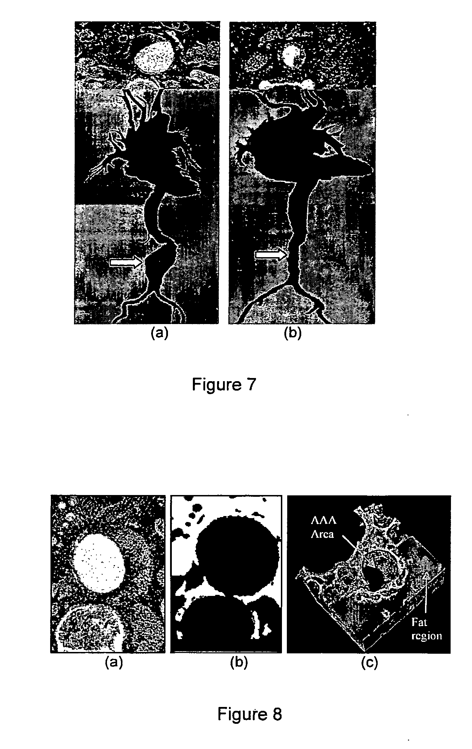

Figure 7 shows examples of the presence of aneurysm that can be identified from the abovementioned features 1-3. In Figure 7a, the projection of the segmented lumen indicates that the lumen is bent under the influence of a large thrombus, and the diameter of the lumen is also enlarged. In Figure 6b, the projection of the segmented lumen in the coronal view does not indicate the presence of the aneurysm, however the cross section in the top image illustrates that the lumen has been disfigured (non-circular) due to the existence of aneurysm. In such cases, the compactness and circularity measures is used to examine the cross sections of the lumen.

If the aneurysm is not detected following the evaluation of the features 1-3, all the objects that are attached to the lumen are first extracted and then examined based on the features that can be obtained from the raw image such as HU attenuations, gradient and texture. These attached objects to the extracted lumen are treated as Potential Regions (PR) and are obtained as given in Equation 3.

$$PR = (L_{\exp} - L) \bigcap \sim (non\_AAA) \qquad\qquad (3)$$

Where $L$ is the extracted lumen and $L_{exp}$ is the expanded (enlarged) lumen, $\cap$ and '$\sim$' are intersection and logical NOT operations respectively. The $non\_AAA$ symbol stands for regions that cannot be part of aneurysm. These regions include fat, spine and blood vessels. Due to their prominent features compared to those of the aneurysm regions, they can easily be separated and hence used as a solid obstacle during subsequent detection and segmentation of aneurysm. Before describing how the potential regions are obtained and evaluated, the description regarding the extraction of the $non\_AAA$ regions is presented.

### 4.1) Fat Regions

Fat regions are darker than the aneurysm and can easily be extracted by using a thresholded segmentation followed by morphological opening operation. In some large size aneurysm, small dark regions might exist, within the aneurysm, that could be natural or due to image acquisition artefact. As regard to this, fat regions with a relatively small size are ignored. The size limit for the fat region identification was found empirically to be $50m^3$. Figure 8 shows an example of an extracted fat region that surrounds the aneurysm area.

### 4.2) Spine receipt

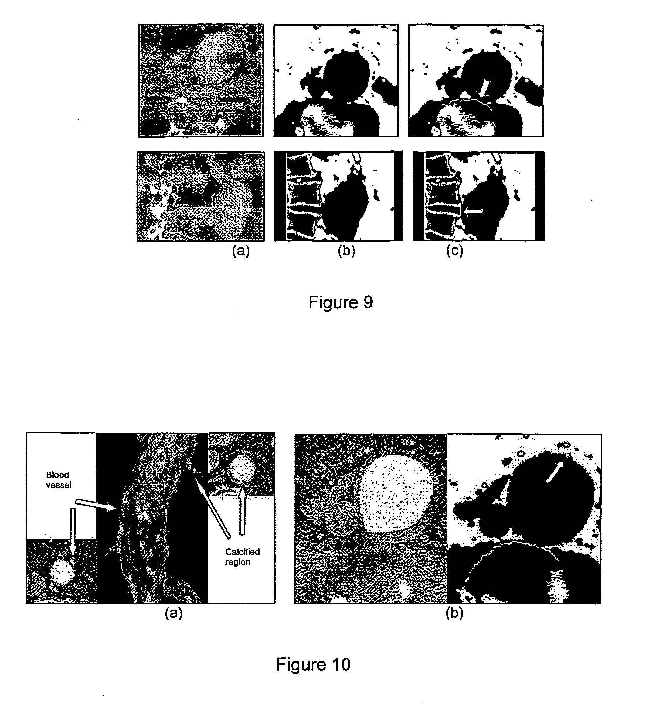

Spine is another object that is located very close to the aorta and hence it is useful to use it as a $non\_AAA$ region. Extracting the spinal bones can easily be done due to their high HU values. However the spine is made of pieces of bones that are held together with a muscle like object called Disk. The Disks have HU values that are similar to that of the thrombus. This leads to the formation of openings in the slices where the Disk regions touch the borders of the aneurysm. These openings (or gaps) can easily be filled by interpolating between the bone edges of the spine, which are located a few slices above and below. Figure 9 shows an example of filling the gap between bone edges of the spine. As can be seen, the aneurysm (the thrombus portion) is touching the spinal Disk and the gap-filled line has clearly separated the thrombus from the Disk region despite the similarity in the HU values.

### 4.3) Blood Vessels

There are several tiny blood vessels that stem out of the lumen. They appear as small circles in consecutive axial images. They can easily be mistaken with the calcified regions when viewed on the axial images. Unlike the calcified regions, the blood vessels are not part of the aneurysm and thus should be included in the non-AAA regions. One prominent feature that can be used to distinguish between the blood vessels and the calcified regions is that the blood vessels have compact and circular cross section that span over a relatively large number of slices. Although calcified regions might have similar contrast and their cross sections might be compact and circular, they are generally small 3D objects with elongated surface that occupy a few slices. Therefore a combination of circularity-compactness and 3D connectivity analysis is used to identify the blood vessels. Figure 10a shows a portion of an aorta where both blood vessel and calcified region exits. The 3D view clearly shows the difference in the geometrical features of the blood vessel and the calcified region. Figure 10b shows the result of blood vessel identification which is added to the non-AAA region.

### 4.4) Potential Regions

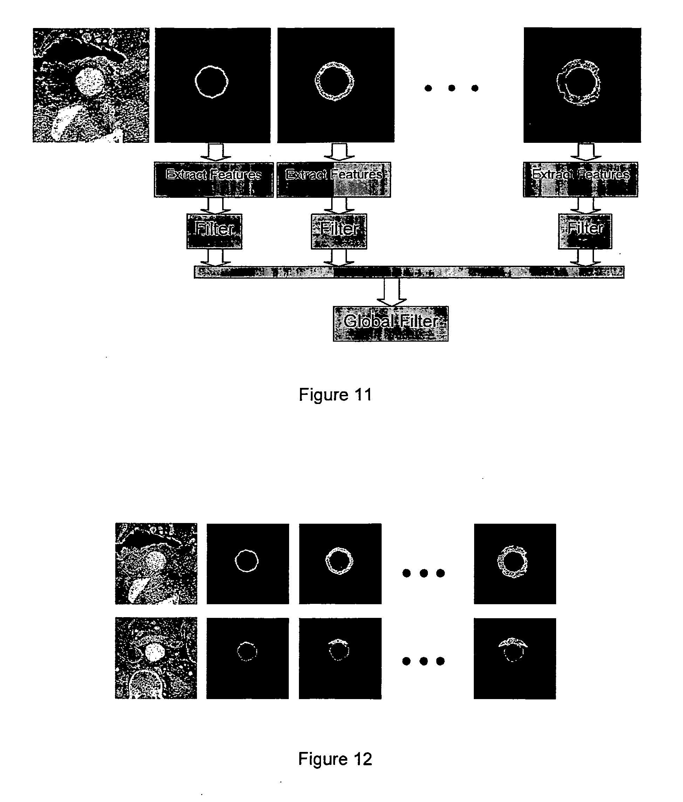

As indicated in Equation 3, the Potential Regions (PRs) are obtained by subtracting the lumen from its expansion and finding the intersection of this result with the inverse of non-AAA regions. The PR regions are then used as a mask to extract the corresponding features from the raw image. The features are fed into a rule-based filter to identify whether the PR contains aneurysm or not. In order to make the process more robust, several expansions of the lumen is used (refer to Equation 1). The PR regions from different expansions are fed to the rule-based filters separately and a global filter is used to make the final decision based on the results obtained from those rule-based filters. This process is shown graphically in Figure 11. The features used for the first filtering stage are intensity, gradient and texture based.

Figure 12 shows a sequence of different lumen expansions and the corresponding attached regions for two cases, one with aneurysm (the top row) and one without (the bottom row).

### 5) Segmentation of Aneurysm

The *non_AAA* regions that were described earlier can be used as a strong barrier (mask) for finding the borders of the aneurysm. This mask is not totally closed up as there are some gaps and openings due to the existence of different tissues touching the aorta with some having similar intensity values as the aneurysm regions. These gaps create leakage path for any region segmentation. To avoid the leakage, an elliptical approximation of the aneurysm region is found first and then fine-tuned using a deformable model.

### 5.1) Ellipsoid Approximation

The distance map of the mask, the *non-AAA* regions, provides useful information of where the entire or parts of the aneurysm might be. The centre point, where the distance value is at its maximum, indicates the deepest part of the aneurysm basin. From this centre point, the core of the region is extracted with 70% of the maximum value; the core region extraction was described above. Note that the higher percentage of the core will create a core region that is more resemblance to the shape of the original region. The sharp edges of this core region are then reduced by applying the opening morphological operation of window size 3x3. This region is then ballooned out until it hits the edges of the mask region. The ballooning process is done by first finding the minimum distance between the edges of the core region and the mask and enlarging the core by the amount equal to the minimum distance value.

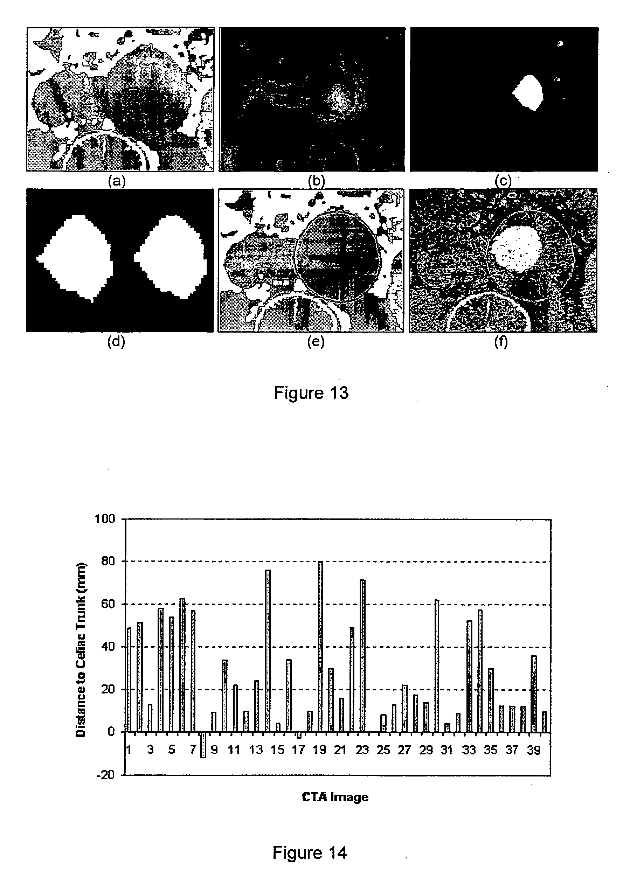

Figure 13 shows an example of the ellipsoid approximation. The 70% core region in Figure 13c is created from the distance map of Figure 13b applied on the *non_AAA* regions mask of Figure 13a. The opening operation has reduced the tiny sharp edges at the bottom and left corner of the core region, as shown in Figure 13d where the regions are zoomed out for better visualization. Figures 13e and 13f clearly shows the strength of this ellipsoid approximation method as it has blocked region from leaking into the relatively larges gaps in the left and bottom of the aneurysm.

### Deformable Model

The ellipsoid approximation provides a very good estimate for defining the borders of the aneurysm. In most cases, the ellipsoid regions cover the exact borders of the aneurysm; in other cases, they would be located very close to the true borders of the aneurysm regions. As regards to this, the deformable model only needs to fine-tune the extend of the ellipsoid region.

### <u>Experimental Results</u>

40 CT Angiography scans were used to test the automatic detection and segmentation that is proposed in this paper. The patients were age between 55 and 85 with 4 female and 36 male. The GE LightSpeed VCT machine was used to obtain the images. Scan parameters were 120 kV, 300 to 400 mA and slice thickness 1.0 to 2.0 mm.

The AAA regions in CT data were annotated by three expert Radiologists. It should be noted that oobtaining a ground truth (i.e. a "gold standard") for defining the border of aneurysm in clinical data is challenging. There can be interobserver and intraobserver differences in manually outlined AAA provided by experts. Consequently, validating the acceptability of any AAA segmentation algorithm is subjective.

### Detect and Extract Lumen

In this experiment the effect of different threshold limits on the detection and extraction of the lumen is shown. Since there was no annotation information for the lumen regions (i.e. it is expensive and time consuming to ask the radiologists to annotate both lumen and borders of aneurysm), one set of parameters were used as a test-bench and other results were compared to it. The test-bench was obtained by selecting a set of parameters that resulted in the correct extraction of the lumens for all 40 images based on the visual judgment of an expert radiologist. The parameters for this test-bench were selected as $T_l$=140 HU and $T_h$=700 HU (see the earlier description above). Table 1 shows the result of trying out different parameters for detection and extraction of the lumen in 40 datasets. The Mean Overlap ($MOv$) is obtained as follows:

$$Ov = 2\frac{V(C) \cap V(T)}{V(C) + V(T)} \qquad (3)$$

$$MOv = \frac{1}{N}\sum_{i}^{N} Ov(i) \qquad (4)$$

Where $Ov$ is the overlap between two objects, $V(C)$ and $V(T)$ are the volume of the current and test object respectively; $N$ is the number of CTA images.

For the results in Table 1, the erosion window size was set kept to 3x3x3 and the threshold values $T_l$ and $T_h$ were changed. The algorithm performed well for $T_l$ > 300 HU when $T_h$ was kept to 700. It failed on one dataset for $T_l$=130 HU which was due to strong presence of spine; i.e. the lumen was detected but attached to the spine. The lower threshold values caused filling more gaps in-between spinal bones (including partial volume effect on the disk portions) thus leading to the formation of fairly strong links between lumen and spine. The second part of Table 1 provides the result of altering the upper threshold value $T_h$. With the lower values for $T_h$ (<500 HU), many points were excluded from the segmentation results for the lumen causing it to break into pieces after passing through the morphological operation (see the description above). Therefore, the algorithm could not find a compact 3D object that had the geometrical feature of the lumen.

Table 1. Detecting and extracting lumen using different threshold limits as compared to the results of using parameters $T_l$=150 HU and $T_h$=600 HU.

| Case | Parameters | Mean Overlap | No. Satisfactory |
|------|-----------|--------------|------------------|
| 1 | $T_l$=120, $T_h$=700 | 0.93 | 38 |
| 2 | $T_l$=130, $T_h$=700 | 0.96 | 39 |
| 3 | $T_l$=135, $T_h$=700 | 0.98 | 40 |
| 4 | $T_l$=145, $T_h$=700 | 0.99 | 40 |
| 5 | $T_l$=155, $T_h$=700 | 0.98 | 40 |
| 6 | $T_l$=140, $T_h$=400 | 0.79 | 36 |
| 7 | $T_l$=140, $T_h$=500 | 0.95 | 39 |
| 8 | $T_l$=140, $T_h$=600 | 0.99 | 40 |
| 9 | $T_l$=140, $T_h$=800 | 0.98 | 40 |
| 10 | $T_l$=140, $T_h$=900 | 0.90 | 39 |

Table 2 shows the effect of the erosion operation on separating lumen from other objects in the segmented image. The results are compared to the test bench with parameters $T_l$=150 HU, $T_h$=600 HU and the erosion window size of 7x7x1 (2D erosion slice by slice). Based on these results, the 2D erosion operation is faster than the 3D operation but the later is more robust for cases in this experiment. As trade off between robustness and speed, the 2D erosion operation of 7x7x1 can be chosen.

Table 2. Effect of erosion window size on the detection and extraction of lumen. Cases 1-3 are 2D slice by slice erosion and cases 4-7 are 3D erosion.

| Case | Win size | Mean Overlap | N. failed | Time (s) |
|------|----------|--------------|-----------|----------|
| 1 | 3x3x1 | 0.92 | 3 | 5 |
| 2 | 5x5x1 | 0.96 | 1 | 5.1 |

(continued)

| Case | Win size | Mean Overlap | N. failed | Time (s) |
|---|---|---|---|---|
| 3 | 9x9X1 | 0.98 | 0 | 5.3 |
| 4 | 3x3x3 | 0.85 | 0 | 7.3 |
| 5 | 5x5x5 | 0.88 | 0 | 8.3 |
| 6 | 7x7x7 | 0.88 | 0 | 13 |
| 7 | 9x9x9 | 0.75 | 0 | 21.7 |

As was described above, a special 3D region labeling is applied on the segmented regions following the morphological erosion operation. This region labeling uses the core of the region cross sections in the consecutive slices to identify the existence of connectivity. The size of the region core depends on the percentage of the maximum value of the distance map. Since the region core is only used to identify the connectivity and not to determine the final size of the lumen, its size does not have significant effect on the result. This was proved in an experiment that was conducted using a range of sizes for the core region between 60% and 99% of the centre point's distance value; i.e. core region extraction was described above. These tests were applied on all the datasets using the parameters $T_f$=140, $T_h$=700 and erosion size of 3x3x3.It was observed that the core sizes of above 70% did not have any significant effect on the result and below 70% some lumens were reduced in size since some cross sections were not added due to lack of connectivity. The algorithm was also tested on with the traditional 3D labeling. With this, the algorithm did not produced satisfactory results for two cases. In one case, the lumen was attached to the spine and in the other case the lumen was connected to one kidney through the renal artery.

## End of Lung Position

This experiment investigates the sensitivity of the algorithm to the parameters $T_{size}$ and $T_{cnt}$ in Equations 1 and 2 to fmd end of the lung location. Table 1 shows the results of finding the end of lung positions using different parameters. The minimum, maximum and average distance, in millimeter, between the end of lung location and the actual position of the Celiac Trunk are given. The latter location was provided by one of the radiologists. The algorithm failed on one data when the size limit for identifying the isolated objects was small ($T_{size}$=3). This is because some of the artifacts from the segmented lung were identified as valid isolated objects. Apart from the choice of small size limit, the algorithm was robust for a range of experimental values; i.e. due to the paper limitation, many of the combinations of the parameters are not shown in Table 3. Out of these results, the parameters of $T_{size}$=6, $T_{cnt}$=6 were chosen as a trade-off between robustness and accuracy.

Table 3. Finding end of lung using different parameter combinations

| Parameters | Min, Max distance to Celiac (mm) | Average distance to Celiac (mm) |
|---|---|---|
| $T_{size}$=3, $T_{cnt}$=3 | -239, 80 | 35.6 |
| $T_{size}$=4, $T_{cnt}$=4 | -159,80 | 33.9 |
| $T_{Size}$=5, $T_{cnt}$=5 | -12, 80 | 30.8 |
| $T_{size}$=6, $T_{cnt}$=6 | -12, 80 | 31.69 |
| $T_{size}$=10, $T_{cnt}$=10 | -4, 100 | 44.7 |
| $T_{size}$=5, $T_{cnt}$=10 | -12, 80 | 33.7 |
| $Tsize$=5, $T_{cnt}$=20 | 6,88 | 48.6 |

Figure 14 shows the distance map between the end of lung location and the Celiac Truck using the parameters $T_{size}$=6, $T_{cnt}$=6 for 40 CT images. As can be seen, in majority of the images, the Celiac Trunk is located after the position of lung that algorithm finds. From this result, it can be safely concluded that the search limit for find the Celiac Trunk automatically can be safely set to 40 mm above and 120 mm below the end of lung position.

**Segmentation of Aneurysm**

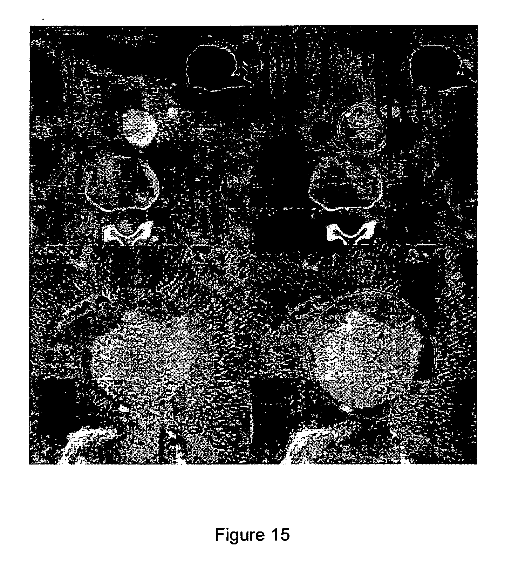

Figure 15 shows examples of the segmentation of AAA.

**References**

[1] PatientPlus: http://www.patient.co.uk/showdoc/40024885/

[2] BUPA: http://hcd2.bupa.co.uk/fact_sheets/html/aortic aneurysm.html

[3] The Mercury News: http://www.mercurynews,com/mld/mercurynews/news/nation/16352304.htm

[4] Jamshid Dehmeshki, Hamdan Amin, Wing Wong, Mandana Ebadian Dehkordi, John Costello, Mary Roddie, "Automatic Identification of Colonic Polyp on High Resolution CT", Presented at the SPIE Int'l Symposium 14 - 19 Feb 2004, Medical Imaging, Town & Country Hotel, San Diego, California; 156- Proc. of SPIE Vol. 5370; pp155-62.

[5] Zhao B, Gamsu G, Ginsberg MS, Jiang L, Schwartz H. Automatic detection of small lung nodules on CT utilizing a local density maximum algorithm. Journal of Applied Clinical Medical Physics 2003; 4:248-260.

[6] R. Ravhon, D. Adam, and L. Zelmanovitch, "Validation of ultrasonic image boundary recognition in abdominal aortic aneurysm," IEEE Trans. Med. Imaging 20_8, 751-763.2001.

[7] Loncaric, M. Subasic, and E. Sorantin, "3-d deformable model for abdominal aortic aneurysm segmentation from ct images," in IWISPA 2000, pp. 139-144.

[8] Subasic, S. Loncaric, and E. Sorantin, "Region-based deformable model for aortic wall segmentation," in ISPA, 2003, Vol. 2, pp. 731-735.

[9] M. de Bruijne, B. van Ginneken, W. J. Niessen, J. B. A. Maintz, and M. A. Viergever, "Active shape model based segmentation of abdominal aortic aneurysms in cta images," Proc. SPIE 4684, 463-474,2002.

[10] M. de Bruijne, B. van Ginneken, M. A. Viergever, and W. J. Niessen, "Interactive segmentation of abdominal aortic aneurysms in CTA data," Med. Image Anal., vol. 8, no. 2, pp. 127-138, 2004.

[11] M. de Bruijne, B. van Ginneken, M. A. Viergever, and W. J. Niessen, "Adapting active shape models for 3d segmentation of tubular structures in medical images," Inf. Process. Med. Imaging 18, 136-147, 2003

[12] A. Giachetti and G. Zanetti, "Aquatics reconstruction software: The design of a diagnostic tool based on computer vision algorithms," Computer Vision and Mathematical Methods in Medical and Biomedical Image Analysis 3117, 48-63, 2004

[13] M. Subasic, S. Loncaric, and E. Sorantin, "3-D image analysis of abdominal aortic aneurism," Proc. SPIE Med. Imag., vol. 4684, pp. 1681-1689, 2002.

[14] S. D. Olabarriaga, J. M. Rouet, M. Fradkin, M. Breeuwer, and W. J. Niessen, "Segmentation of thrombus in abdominal aortic aneurysms from cta with nonparametric statistical grey level appearance modeling," IEEE Trans. Med. Imaging 24_4_, 477-485, 2005.

[15] Zhuge F, Rubin GD, Sun S, Napel S "An abdominal aortic aneurysm segmentation method: level set with region and statistical information." Med Phys 2006; 33: 5: 1440-53

**Commercial applications:**

[4b] Lung-Siemens: http://www.medical.siemens.com/webapp/wcs/stores/servlet/PressReleaseView~q_catalogId~ e_- -1~a_catTree~e_null~a-langId~e= 1~a_ageId_77320~a_storeId~e-10001.htm

[5b] GE-Mammography http://www.gehealthcare.com/rad/whc/products/mswh2OOOd.html# 1 bis

**Some of the medical imaging scientific papers by members of Mediar Ltd:**

*Heart:*

[6b] J. Dehmeshki, X. Ye, H. Amin, M. Abaei, X. Lin, S.D. Qanadli, "Volumetric Quantification of Atherosclerotic Plaque in CT Considering Partial Volume Effect" in 'IEEE Transactions on Medical Imaging', March 2007, pp: 273-282

*Brain:*

[7b] J. Dehmeshki, D.T. Chard, S.M. Leary, H.C. Watt, N.C. Silver, P.S. Tofts, A.J. Thompson, D.H. Miller, "The Normal Appearing Grey Matter in Primary Progressive Multiple Sclerosis. A Magnetisation Transfer Imaging Study" in 'Journal of Neurology', 250(1) January, (2003)

### *Colon:*

[8b] Jamshid Dehmeshki, Hamdan Amin, Wing Wong, Mandana Ebadian Dehkordi, John Costello, Mary Roddie, "Automatic Identification of Colonic Polyp on High Resolution CT", Presented at the SPIE Int'l Symposium 14 - 19 Feb 2004, Medical Imaging, Town & Country Hotel, San Diego, California; 156- Proc. of SPIE Vol. 5370; pp155-62.
[9b] A Taylor, S. Halligan, D. Burling, M.E. Roddie, L. Honeyfield, H. Amin, J. Dehmeshki, "Computer Assisted Reader Software Versus Expert Reviewers for Polyp Detection on CT Colonography" in 'American Journal of Roentgenology', 186, pp. 696-702. (2006).

### *Lung:*

[10b] Dehmeshki, J.; Ye, X.; Costello, J, "Shape based region growing using derivatives of 3D medical images: application to semiautomated detection of pulmonary nodules", ICIP 2003 Proceedings, International Conference on Image Processing, Volume 1, Issue , 14-17 7 Sept. 2003 Page(s): I - 1085-8 vol. 1

### **Academic papers on AAA:**

[11b] R. Ravhon, D. Adam, and L. Zelmanovitch, "Validation of ultrasonic image boundary recognition in abdominal aortic aneurysm," IEEE Trans. Med. Imaging 20_8, 751-763.2001.
[12b] Loncaric, M. Subasic, and E. Sorantin, "3-d deformable model for abdominal aortic aneurysm segmentation from ct images," in IWISPA 2000, pp. 139-144.
[13b] Subasic, S. Loncaric, and E. Sorantin, "Region-based deformable model for aortic wall segmentation," in ISPA, 2003, Vol. 2, pp. 731-735.
[14b] M. de Bruijne, B. van Ginneken, W. J. Niessen, J. B. A. Maintz, and M. A. Viergever, "Active shape model based segmentation of abdominal aortic aneurysms in cta images," Proc. SPIE 4684, 463-474,2002.
[15b] M. de Bruijne, B. van Ginneken, M. A. Viergever, and W. J. Niessen, "Interactive segmentation of abdominal aortic aneurysms in CTA data," Med. Image Anal., vol. 8, no. 2, pp. 127-138, 2004.
[16b] M. de Bruijne, B. van Ginneken, M. A. Viergever, and W. J. Niessen, "Adapting active shape models for 3d segmentation of tubular structures in medical images," Inf. Process. Med. Imaging 18, 136-147, 2003
[17b] A. Giachetti and G. Zanetti, "Aquatics reconstruction software: The design of a diagnostic tool based on computer vision algorithms," Computer Vision and Mathematical Methods in Medical and Biomedical Image Analysis 3117, 48-63, 2004
[18b] M. Subasic, S. Loncaric, and E. Sorantin, "3-D image analysis of abdominal aortic aneurism," Proc. SPIE Med. Imag., vol. 4684, pp. 1681-1689, 2002.
[19b] S. D. Olabarriaga, J. M. Rouet, M. Fradkin, M. Breeuwer, and W. J. Niessen, "Segmentation of thrombus in abdominal aortic aneurysms from cta with nonparametric statistical grey level appearance modeling," IEEE Trans. Med. Imaging 24_4_, 477-485, 2005.
[20b] Zhuge F, Rubin GD, Sun S, Napel S "An abdominal aortic aneurysm segmentation method: level set with region and statistical information." Med Phys 2006; 33: 5: 1440-53

## Claims

1. A computer-implemented, automated method of segmenting computed tomography angiography (CTA) images, comprising the following steps:

   (1) receiving said CTA images, identifying the lumen of an aorta in the received images, and extracting said lumen, this step comprising the following sub-steps:

   a. segmenting said CTA images using threshold limits that represent the intensities of the contrast agent intensity range to obtain segmented CTA images;
   b. performing a morphological erosion operation to break connections between 3D objects in said segmented CTA images;
   c. applying a geometrical filter to identify the lumen among a plurality of 3D objects;

(2) identifying the abdominal portion of said extracted lumen in said CTA images;

(3) performing a straightening of said abdominal portion to obtain a straightened abdominal portion;

(4) searching for features of said straightened abdominal portion that indicate the presence of an aneurysm;

(5) if the presence of an aneurysm is detected based on said features, segmenting the aneurysm using said extracted lumen as an initial surface of a deformable model.

2. The method as defined in claim 1, wherein step (2) comprises the following sub-steps:

   a. finding the lower end location of the lung region;

   b. finding the celiac trunk;

   c. finding the iliac arteries junction using a smoothed centreline and cluster connectivity analysis comprising the following steps:

   i. getting the centreline using the Distant Transform-based Skeletonization method;

   ii. smoothing the centreline by using an iterative method in which the current location of a voxel within the centreline is updated by the weighted average of deviation vector of its neighbour voxels;

   iii. creating the cluster connectivity class of the centreline;

   iv. identifying the main branches, i.e. the iliac arteries, by evaluating the length of each branch from the cluster connectivity class.

3. The method as defined in claim 2, wherein finding the lower end location of the lung region is made by using the following image processing algorithms slice by slice until no more lung region is found:

   i. performing an adaptive fuzzy thresholding segmentation algorithm on said segmented CTA images;

   ii. extracting the possible lung regions by finding holes originating from air volumes within each segmented image, the hole extraction being done by applying the flood-fill algorithm on the segmented image and subtracting the result from the segmented image;

   iii. checking if the region obtained in step ii contains a plurality of holes originating from a cross sectional view of blood vessels within the lung space;

   iv. if lung region is identified in step iii, then going to step i examining the next slice in downward direction; otherwise the current slice being the lower end location of the lung regions.

4. The method as defined in claim 2, wherein finding the celiac trunk using the lower end of lung location and the extracted lumen comprises the following steps;

   i. creating a sub-image as a search volume whose depth is limited to 50 mm above and 100 mm below the lower end of lung location;

   ii. segmenting the sub-image and keeping the region that intersects with the lumen;

   iii. projecting all voxels in the sagittal view into one slice to obtain a projection image;

   iv. performing an erosion operation of a window size 15x15 followed by a dilation operation of 19x19 window size to obtain an erosion image;

   v. subtracting the erosion image obtained in step iv from the projection image obtained in step iii, which will result in several isolated objects that represent the branches of the lumen;

   vi. getting the first isolated object from the left and top as the first branch of the lumen, which is the celiac trunk.

5. The method as defined in any one of the preceding claims, comprising the steps of using the centreline and the location of the celiac trunk and iliac arteries as end points,straightening the centreline by finding the shortest path between the two end points such that the geometrical mapping that is obtained from the centreline transformation can be applied on the segmented or the original image.

6. The method as defined in any one of the preceding claims, further comprising the step of determining a diameter of said straightened abdominal portion.

7. The method as defined in claim 6, comprising the following steps:

   a. checking if said straightened abdominal portion is bent;

   b. checking if the diameter of said straightened abdominal portion is enlarged;

   c. checking if the cross section of said straightened abdominal portion is irregular;

d. checking if the objects attached to said straightened abdominal portion have features of aneurysm, wherein the search for aneurysm among the attached objects comprises excluding non-abdominal-aortic-aneurysm regions obtained by applying the following sub-steps:

    i. getting the non-abdominal-aortic-aneurysm fat regions;
    ii. getting the non-abdominal-aortic-aneurysm high intensity objects;
    iii. getting the non-abdominal-aortic-aneurysm spinal bone gap filling;
    iv. extracting attached objects.

8. The method as defined in claim 7, comprising the steps of:

    - ) using the extracted lumen as an initial surface and said non-abdominal-aortic-aneurysm regions as barriers for a deformable model based segmentation,
    - ) approximating the abdominal-aortic-aneurysm region by robustly fitting a 3-D ellipsoid anisotropic Gaussian-based intensity model to the border of the non-abdominal-aortic-aneurysm regions;
    - ) using a union of this region and the extracted lumen as an initial region to be refined at its border.

9. The method as defined in any one of the preceding claims, further comprising the step of measuring a diameter and/or volume of said aneurysm.

**Patentansprüche**

1. Computer-implementiertes, automatisches Verfahren zur Segmentierung von Computertomographie-Angiographie-(CTA)-Bildern, umfassend die folgenden Schritte:

(1) Erfassen der besagten CTA-Bilder, Identifizieren des Lumens einer Aorta in den erfassten Bildern und Extrahieren des besagten Lumens, wobei dieser Schritt die folgenden Unterschritte umfasst:

    a. Segmentieren der besagten CTA-Bilder unter Verwendung von Schwellenwerten, welche die Intensitäten des Kontrastmittelintensitätsbereiches darstellen, um segmentierte CTA-Bilder zu erhalten;
    b. Durchführen eines morphologischen Erosionsvorganges, um Verbindungen zwischen 3D-Objekten in besagten segmentierten CTA-Bildern aufzutrennen;
    c. Anwenden eines geometrischen Filters zur Identifikation des Lumens entlang einer Vielzahl von 3D-Objekten;

(2) Identifizieren des abdominellen Abschnittes des besagten extrahierten Lumens in den besagten CTA-Bildern;
(3) Durchführen einer Begradigung des besagten abdominellen Abschnittes, um einen begradigten abdominellen Abschnitt zu erhalten;
(4) Suchen nach Merkmalen von besagtem abdominellen Abschnitt, welche auf das Vorliegen eines Aneurysmas hinweisen;
(5) falls das Vorliegen eines Aneurysmas aufgrund der besagten Merkmale nachgewiesen wurde, Segmentieren des Aneurysmas unter Verwendung des besagten extrahierten Lumens als initiale Oberfläche eines deformierbaren Modelles.

2. Verfahren nach Anspruch 1, wobei der Schritt (2) die folgenden Unterschritte umfasst:

    a. Auffinden der unteren Endstelle des Lungenbereiches;
    b. Auffinden des Bauchraumes;
    c. Auffinden der Verzweigung der Beckenarterie unter Verwendung einer geglätteten Mittellinie und einer Cluster-Konnektivitätsanalyse, umfassend die folgenden Schritte:

        i. Bestimmen der Mittellinie unter Verwendung der Distanztransformationbasierten Skelettierungsmethode;
        ii. Glätten der Mittellinie unter Verwendung eines iterativen Verfahrens, wobei die aktuelle Lage eines Voxels innerhalb der Mittellinie um den gewichteten Mittelwert des Abweichungsvektors des benachbarten Voxels aktualisiert wird;
        iii. Erstellen der Cluster-Konnektivitätsklasse der Mittellinie;
        iv. Identifizieren der Hauptverzweigungen, z.B. der Beckenarterien, durch Ermitteln der Länge eines jeden Zweigs aus der Cluster-Konnektivitätsklasse.

**3.** Verfahren nach Anspruch 2, wobei das Auffinden der unteren Endstelle des Lungenbereiches unter Verwendung der folgenden Bildverarbeitungsalgorithmen Schnitt um Schnitt bis kein weiterer Lungenbereich gefunden wird, durchgeführt wird:

i. Durchführen eines adaptiven Fuzzy-Schwellenwert-Segmentationsalgorithmus auf die besagten segmentierten CTA-Bilder;
ii. Extrahieren der möglichen Lungenbereiche durch Auffinden von Löchern, die von Luftvolumina stammen, innerhalb eines jeden segmentierten Bildes, wobei die Lochextraktion durch Anwenden des Flood-fill-Algorithmus auf das segmentierte Bild und Subtrahieren des Ergebnisses vom segmentierten Bild ausgeführt wird;
iii. Überprüfen, ob die in Schritt ii erhaltenen Bereiche eine Vielzahl von Löchern, die von einer Querschnittsansicht von Blutgefässen innerhalb des Lungenraums stammen, enthalten;
iv. falls im Schritt iii ein Lungenbereich identifiziert wird, dann wird zum Schritt i übergegangen, in welchem der nächste Schnitt in Abwärtsrichtung überprüft wird; andernfalls ist der aktuelle Schnitt die untere Endstelle des Lungenbereiches.

**4.** Verfahren nach Anspruch 2, wobei das Auffinden des Bauchraumes unter Verwendung des unteren Endes der Lungenstelle und des extrahierten Lumens die folgenden Schritte umfasst;

i. Erstellen eines Teilbildes als Suchvolumen, dessen Tiefe auf bis zu 50 mm oberhalb und 100 mm unterhalb der unteren Endstelle des Lungenbereichs beschränkt ist;
ii. Segmentieren des Teilbildes und Beibehalten desjenigen Bereiches, welcher das Lumen schneidet;
iii. Projizieren aller Voxel in sagittaler Ansicht in einen Schnitt, um ein Projektionsbild zu erhalten;
iv. Durchführen eines Erosionsvorganges auf eine Fenstergrösse von 15x15, gefolgt von einem Dilatationsvorgang auf eine Fenstergrösse von 19x19, um ein Erosionsbild zu erhalten;
v. Subtrahieren des im Schritt iv erhaltenen Erosionsbildes von dem im Schritt iii erhaltenen Projektionsbild, woraus sich mehrere isolierte Objekte ergeben, welche die Zweige des Lumens darstellen;
vi. Zuordnen des ersten isolierten Objektes von links und von oben als der erste Zweig des Lumens, welcher der Bauchraum ist.

**5.** Verfahren nach einem der vorangegangenen Ansprüche, umfassend die Schritte des Verwendens der Mittellinie und der Lage des Bauchraumes und der Beckenarterien als Endpunkte, des Begradigens der Mittellinie durch Auffinden des kürzesten Weges zwischen den beiden Endpunkten derart, dass die aus der Mitellinientransformation erhaltene geometrische Abbildung auf das segmentierte oder das Originalbild angwendet werden kann.

**6.** Verfahren nach einem der vorangegangenen Ansprüche, weiterhin umfassend den Schritt des Bestimmens eines Durchmessers des besagten begradigten abdominellen Abschnitts.

**7.** Verfahren nach Anspruch 6, umfassend die folgenden Schritte:

a. Überprüfen, ob der besagte begradigte abdominelle Abschnitt gebogen ist;
b. Überprüfen, ob der Durchmesser des besagten begradigten abdominellen Abschnitts aufgeweitet ist;
c. Überprüfen, ob der Querschnitt des besagten begradigten abdominellen Abschnitts unregelmässig ist;
d. Überprüfen, ob die mit dem besagten begradigten abdominellen Abschnitt verbundenen Objekte Merkmale eines Aneurysmas aufweisen, wobei die Suche nach Aneurysmen unter den verbundenen Objekten den Ausschluss von durch Anwendung der folgenden Teilschritte erhaltenen, nicht-abdominell-Aortaaneurysmabereiche umfasst:

i. Bestimmen der nicht-abdominell-Aortaaneurysma-Fettbereiche;
ii. Bestimmen der nicht-abdominell-Aortaaneurysma-Objekte mit hoher Intensität;
iii. Bestimmen der nicht-abdominell-Aortaaneurysma-Wirbelsäulen-Knochenspaltfiillung;
iv. Extrahieren der verbundenen Objekte.

**8.** Verfahren nach Anspruch 7, umfassend die Schritte:

- ) Verwendung des extrahierten Lumens als eine initiale Oberfläche und der besagten nicht-abdominell-Aortaaneurysmabereiche als Barrieren für eine Segmentation gemäss einem deformierbaren Modell,

- ) Approximieren des abdominell-Aortaaneurysmabereichs durch eine robuste Angleichung eines 3-D-ellipsoiden anisotropen Gaussbasierten Intensitätsmodelles an die Ränder der nicht-abdominell-Aortaaneurysmabereiche;
- ) Verwenden einer Verbindung dieses Bereiches und des extrahierten Lumens als ein initialer, an seinen Rändern zu verfeinernder Bereich.

9. Verfahren nach einem der vorangegangenen Ansprüche, weiterhin umfassend den Schritt des Messens eines Duchmessers und/oder eines Volumens des besagten Aneurysmas.

**Revendications**

1. Procédé automatisé exécuté par ordinateur de segmentation d'images d'angiographie par tomodensitométrie (angio-scanner-CTA) comprenant les étapes consistant à :

   (1) recevoir les images CTA, identifier le lumen de l'aorte dans les images reçues et extraire ce lumen, cette étape renfermant les sous-étapes consistant à :

      a. segmenter les images CTA en utilisant des limites de seuil qui représentent les intensités de la plage d'intensités de l'agent de contraste pour obtenir des images CTA segmentées,
      b. exécuter une opération d'érosion morphologique pour rompre des liaisons entre des objets 3D dans les images CTA segmentées,
      c. appliquer un filtre géométrique pour identifier le lumen parmi un ensemble d'objets 3D,

   (2) identifier la partie abdominale du lumen extrait dans les images CTA,
   (3) exécuter un redressement de la partie abdominale pour obtenir une partie abdominale redressée,
   (4) rechercher des caractéristiques de cette partie abdominale redressée qui indiquent la présence d'un anévrisme,
   (5) si la présence d'un anévrisme est détectée à partir de ces caractéristiques, segmenter l'anévrisme en utilisant le lumen extrait comme surface initiale d'un modèle déformable.

2. Procédé conforme à la revendication 1, selon lequel l'étape (2) comporte les sous étapes consistant à :

      a. trouver l'extrémité inférieure de la région pulmonaire,
      b. trouver le tronc coeliaque,
      c. trouver la jonction des artères iliaques en utilisant un axe lissé et l'analyse des connexions typologiques comprenant les étapes consistant à :

         i. obtenir l'axe en utilisant la méthode de squelettisation basée sur la transformation de la distance,
         ii. lisser l'axe en utilisant une méthode d'itération dans laquelle la localisation réelle d'un voxel dans l'axe central est actualisée par la moyenne pondérée du vecteur de déviation des voxels voisins,
         iii. créer une classe de connexion typologique de l'axe,
         iv. identifier les branches principales, c'est-à-dire les artères iliaques en évaluant la longueur de chaque branche à partir de la classe de connexion typologique.

3. Procédé conforme à la revendication 2, selon lequel l'étape consistant à trouver la position de l'extrémité inférieure de la région pulmonaire est mise en oeuvre en utilisant les algorithmes de traitement d'images ci-dessous, tranche par tranche, jusqu'à ce que l'on ne trouve plus de région pulmonaire :

      i. exécuter un algorithme de segmentation de seuil de flou adaptatif sur les images CTA segmentées,
      ii. extraire les régions pulmonaires possibles en trouvant des vides ayant pour origine des volumes d'air à l'intérieur de chacune des images segmentées, l'extraction des vides étant effectuée en appliquant l'algorithme de remplissage de diffusion sur l'image segmentée et en soustrayant le résultat de l'image segmentée,
      iii. contrôler si la région obtenue dans l'étape ii contient un ensemble de vides provenant d'une section transversale de vaisseaux sanguins à l'intérieur de l'espace pulmonaire,
      iv. si la région pulmonaire est identifiée dans l'étape ii, aller à l'étape i pour examiner la tranche suivante en allant vers le bas ; sinon, la tranche considérée correspond à la position de l'extrémité inférieure de la région pulmonaire.

**4.** Procédé conforme à la revendication 2, selon lequel l'étape consistant à trouver le tronc coeliaque en utilisant l'extrémité inférieure de la position des poumons et le lumen extrait comprend les étapes consistant à :

i. créer une sous-image constituant un volume de recherche dont la profondeur est limitée à 50 mm au-dessus et à 100 mm au-dessous de l'extrémité inférieure de la position des poumons,
ii. segmenter la sous-image et conserver la région qui croise le lumen,
iii. projeter tous les voxels en vue sagittale dans une tranche pour obtenir une image de projection,
iv. exécuter une opération d'érosion d'une dimension de fenêtre de 15 x 15 suivie d'une opération de dilation d'une dimension de fenêtre de 19 x 19 pour obtenir une image d'érosion,
v. soustraire l'image d'érosion obtenue dans l'étape iv de l'image de projection obtenue dans l'étape iii, d'où il résulte une série d'objets isolés qui représentent les branches du lumen,
vi. obtenir le premier objet isolé à partir de la gauche et du haut comme constituant la première branche du lumen qui est le tronc coeliaque.

**5.** Procédé conforme à l'une quelconque des revendications précédentes comprenant les étapes consistant à utiliser l'axe et la position du tronc coeliaque et des artères iliaques comme points finaux, à redresser l'axe en trouvant le chemin le plus court entre les deux points terminaux de sorte que la représentation géométrique obtenue à partir de la transformation de l'axe puisse être appliquée sur l'image segmentée ou l'image originale.

**6.** Procédé conforme à l'une quelconque des revendications précédentes comprenant en outre l'étape consistant à déterminer le diamètre de la partie abdominale redressée.

**7.** Procédé conforme à la revendication 6, comprenant les étapes consistant à :

a. vérifier si la partie abdominale redressée est courbée,
b. vérifier si le diamètre de la partie abdominale redressée est élargi,
c. vérifier si la section transversale de la partie abdominale redressée est irrégulière,
d. vérifier si les objets liés à la partie abdominale redressée ont des caractéristiques d'anévrisme, la recherche d'anévrisme parmi les objets liés comprenant l'exclusion des régions d'anévrisme de l'aorte non-abdominale obtenue en appliquant les sous-étapes consistant à :

i. obtenir les régions grasses d'anévrisme de l'aorte non-abdominale,
ii. obtenir les objets de forte intensité d'anévrisme de l'aorte non-abdominale,
iii. obtenir les remplissages des lacunes entre les vertèbres d'anévrismes de l'aorte non-abdominale,
iv. extraire les objets concernés.

**8.** Procédé conforme à la revendication 7, comprenant les étapes consistant à :

- utiliser le lumen extrait comme surface initiale et les régions d'anévrismes de l'aorte non-abdominale en tant que barrières pour une segmentation basée sur un modèle déformable,
- faire une approximation de la région d'anévrisme de l'aorte abdominale en appliquant étroitement un modèle d'intensité basé sur une gaussienne présentant une anisotropie ellipsoïdale 3D à la limite des régions d'anévrisme de l'aorte non-abdominale,
- utiliser la réunion de cette région et du lumen extrait comme région initiale devant être affinée à ses limites.

**9.** Procédé conforme à l'une quelconque des revendications précédentes, comprenant en outre l'étape consistant à mesurer le diamètre et/ou le volume de l'anévrisme.

```
          ┌─────────────────────┐
          │   Pre-Processing    │
          └─────────────────────┘
                     │
                     ▼
       ┌───────────────────────────┐
       │  Detect and Extract Lumen │
       └───────────────────────────┘
                     │
                     ▼
       ┌───────────────────────────┐
       │     Identify Abdominal    │
       │       Aortic Section      │
       └───────────────────────────┘
                     │
                     ▼
     ┌───────────────────────────────┐
     │   Geometrical Transformation  │
     └───────────────────────────────┘
                     │
                     ▼
              ◇───────────────◇
             ╱    Aneurysm     ╲
             ╲    exists?      ╱
              ◇───────────────◇
                     │ yes
                     ▼
          ┌─────────────────────┐
          │     Segment AAA     │
          └─────────────────────┘
```

Figure 1

(a)          (b)          (c)          (d)

Figure 2

Figure 3

Figure 4

Figure 5

Figure 6

(a)       (b)

Figure 7

(a)       (b)       (c)

Figure 8

(a)　　　　(b)　　　　(c)

Figure 9

(a)　　　　　　　　(b)

Figure 10

Figure 11

Figure 12

Figure 13

Figure 14

Figure 15

**EP 2 279 490 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03075209 A
- WO 2004081874 A
- US 2006025674 A


**Non-patent literature cited in the description**

- **F. ZHUGE et al.** *Med. Phys.,* May 2006, vol. 33 (5), 1440-1453
- **KANITSAR et al.** *Proc. IEE Visualization,* 27 October 2002, 37-44
- Automatic Identification of Colonic Polyp on High Resolution CT. **JAMSHID DEHMESHKI ; HAMDAN AMIN ; WING WONG ; MANDANA EBADIAN DEHKORDI ; JOHN COSTELLO ; MARY RODDIE.** SPIE Int'l Symposium. Medical Imaging, Town & Country Hotel, 14 February 2004, vol. 5370, 155-62
- **ZHAO B ; GAMSU G ; GINSBERG MS ; JIANG L ; SCHWARTZ H.** Automatic detection of small lung nodules on CT utilizing a local density maximum algorithm. *Journal of Applied Clinical Medical Physics,* 2003, vol. 4, 248-260
- **R. RAVHON ; D. ADAM ; L. ZELMANOVITCH.** Validation of ultrasonic image boundary recognition in abdominal aortic aneurysm. *IEEE Trans. Med. Imaging,* 2003, vol. 20 (8), 751-763
- **LONCARIC, M. ; SUBASIC ; E. SORANTIN.** 3-d deformable model for abdominal aortic aneurysm segmentation from ct images. *IWISPA,* 2000, 139-144
- **SUBASIC, S. LONCARIC ; E. SORANTIN.** Region-based deformable model for aortic wall segmentation. *ISPA,* 2003, vol. 2, 731-735
- **M. DE BRUIJNE ; B. VAN GINNEKEN ; W. J. NIESSEN ; J. B. A. MAINTZ ; M. A. VIERGEVER.** Active shape model based segmentation of abdominal aortic aneurysms in cta images. *Proc. SPIE,* 2002, vol. 4684, 463-474
- **M. DE BRUIJNE ; B. VAN GINNEKEN ; M. A. VIERGEVER ; W. J. NIESSEN.** Interactive segmentation of abdominal aortic aneurysms in CTA data. *Med. Image Anal.,* 2004, vol. 8 (2), 127-138
- **M. DE BRUIJNE ; B. VAN GINNEKEN ; M. A. VIERGEVER ; W. J. NIESSEN.** Adapting active shape models for 3d segmentation of tubular structures in medical images. *Inf. Process. Med. Imaging,* 2003, vol. 18, 136-147
- **A. GIACHETTI ; G. ZANETTI.** Aquatics reconstruction software: The design of a diagnostic tool based on computer vision algorithms. *Computer Vision and Mathematical Methods in Medical and Biomedical Image Analysis,* 2004, vol. 3117, 48-63
- **M. SUBASIC ; S. LONCARIC ; E. SORANTIN.** 3-D image analysis of abdominal aortic aneurism. *Proc. SPIE Med. Imag.,* 2002, vol. 4684, 1681-1689
- **S. D. OLABARRIAGA ; J. M. ROUET ; M. FRADKIN ; M. BREEUWER ; W. J. NIESSEN.** Segmentation of thrombus in abdominal aortic aneurysms from cta with nonparametric statistical grey level appearance modeling. *IEEE Trans. Med. Imaging,* 2005, vol. 24 (4), 477-485
- **ZHUGE F ; RUBIN GD ; SUN S ; NAPEL S.** An abdominal aortic aneurysm segmentation method: level set with region and statistical information. *Med Phys,* 2006, vol. 33 (5), 1440-53
- **J. DEHMESHKI ; X. YE ; H. AMIN ; M. ABAEI ; X. LIN ; S.D. QANADLI.** Volumetric Quantification of Atherosclerotic Plaque in CT Considering Partial Volume Effect. *IEEE Transactions on Medical Imaging,* March 2007, 273-282
- **J. DEHMESHKI ; D.T. CHARD ; S.M. LEARY ; H.C. WATT ; N.C. SILVER ; P.S. TOFTS ; A.J. THOMPSON ; D.H. MILLER.** The Normal Appearing Grey Matter in Primary Progressive Multiple Sclerosis. A Magnetisation Transfer Imaging Study. *Journal of Neurology,* January 2003, vol. 250 (1
- **A TAYLOR ; S. HALLIGAN ; D. BURLING ; M.E. RODDIE ; L. HONEYFIELD ; H. AMIN ; J. DEHMESHKI.** Computer Assisted Reader Software Versus Expert Reviewers for Polyp Detection on CT Colonography. *American Journal of Roentgenology,* 2006, vol. 186, 696-702
- **DEHMESHKI, J. ; YE, X. ; COSTELLO, J.** Shape based region growing using derivatives of 3D medical images: application to semiautomated detection of pulmonary nodules. *ICIP 2003 Proceedings, International Conference on Image Processing,* 07 September 2003, vol. 1 (14-17), I - 1085-8

- **R. RAVHON ; D. ADAM ; L. ZELMANOVITCH.** Validation of ultrasonic image boundary recognition in abdominal aortic aneurysm. *IEEE Trans. Med. Imaging,* 2001, vol. 20 (8), 751-763

- **LONCARIC, M. SUBASIC ; E. SORANTIN.** 3-d deformable model for abdominal aortic aneurysm segmentation from ct images. *IWISPA,* 2000, 139-144